# FASCICULE DE BREVET EUROPEEN

(11) **EP 2 440 509 B1**
(45) Date de publication et mention de la délivrance du brevet: **27.04.2016**
(21) Numéro de dépôt: 10727102.5
(22) Date de dépôt: 05.05.2010
(51) Int. Cl.: C07C 17/354, C07C 19/08

(54) **PROCEDE DE FABRICATION DU HEXAFLUOROPROPANE**
VERFAHREN ZUR HERSTELLUNG VON HEXAFLUORPROPAN
METHOD FOR PRODUCING HEXAFLUOROPROPANE

(30) Priorité: 12.06.2009 FR 0953941
(43) Date de publication de la demande: 18.04.2012
(73) Titulaire: Arkema France, 92700 Colombes (FR)
(72) Inventeur: DEVIC, Michel, F-69110 Sainte Foy Les Lyon (FR); DOUCET, Nicolas, F-69007 Lyon (FR); WENDLINGER, Laurent, F-69510 Soucieu En Jarrest (FR); CAVALLINI, Géraldine, F-69360 Saint-symphorien D'ozon (FR)
(74) Mandataire: Dang, Doris
(86) Numéro de dépôt international: PCT/FR2010/050859
(87) Numéro de publication internationale: WO 2010/142877

(56) Documents cités:
- EP-A1- 1 916 232
- DATABASE WPI Week 200872 Thomson Scientific, London, GB; AN 2008-M14458 XP002564309 & CN 101 148 395 A (ZHEJIANG LANTIAN ENVIRONMENTAL PROTECTIO) 26 mars 2008 (2008-03-26)
- DATABASE CA [Online] CHEMICAL ABSTRACTS SERVICE, COLUMBUS, OHIO, US; 1961, KNUNYANTS, I. L. ET AL: "Reactions of fluoro olefins. XIII. Catalytic hydrogenation of perfluoro olefins" XP002564154 extrait de STN Database accession no. 1961:2127 cité dans la demande & KNUNYANTS, I. L. ET AL: "Reactions of fluoro olefins. XIII. Catalytic hydrogenation of perfluoro olefins" IZVESTIYA AKADEMII NAUK SSSR, SERIYA KHIMICHESKAYA 1412-18 CODEN: IASKA6; ISSN: 0002-3353, 1960,

## Description

La présente invention concerne un procédé de fabrication du 1,1,1,2,3,3-hexafluoropropane par hydrogénation de l'hexafluoropropène.

Le document de Knunyants et al., Journal of the USSR Academy of Sciences, Chemistry Department, «reactions of fluoro-olefins», report 13, "catalytic hydrogénation of perfluoro-olefins", 1960, décrit l'hydrogénation sensiblement quantitative de l'hexafluoropropène (HFP) sur un catalyseur à base de palladium supporté sur l'alumine, la température passant de 20°C à 50°C, puis étant maintenue à cette valeur.

Le 1,1,1,2,3,3-hexafluoropropane est utile pour la fabrication du 1,2,3,3,3-pentafluoropropène.

Les essais de l'art antérieur précité ont été effectués à l'échelle laboratoire et les documents sont totalement muets sur la durée de vie de ces catalyseurs.

La réaction d'hydrogénation telles que décrite précédemment est fortement exothermique et pose des problèmes à l'échelle industrielle. Cette forte exothermicité est néfaste à la durée de vie du catalyseur.

La présence d'un composé, autre que les réactifs, dans le flux réactionnel peut également être à l'origine d'une désactivation rapide du catalyseur.

Par ailleurs, le document EP 1916232 propose une réaction d'hydrogénation multiétagée d'un composé oléfinique pour obtenir une conversion et une sélectivité élevée. L'exemple 1 décrit la réaction d'hydrogénation étagée de l'hexafluoropropène en présence d'un catalyseur supporté sur charbon dans quatre réacteurs avec une température du flux gazeux à la sortie du premier réacteur de 66°C, une température de 104°C à la sortie du deuxième réacteur (pour une conversion de 40%), une température de 173°C à la sortie du troisième, une température de 100°C à la sortie du quatrième. Il est prévu des étapes de refroidissement entre les réacteurs avec une température du premier bain de 55°C et celle du deuxième bain de 111 °C.

Le procédé tel que décrit dans le document EP 1916232 est coûteux en investissement et en outre, sa mise en oeuvre n'est pas aisée.

Le procédé, selon la présente invention, permet de maîtriser l'exothermicité de la réaction d'hydrogénation tout en gardant une très bonne conversion et sélectivité et/ou réduire la désactivation du catalyseur.

Le procédé selon la présente invention est caractérisé en ce que (i) l'on fait réagir en phase gazeuse de l'hexafluoropropène avec de l'hydrogène en quantité surstoechiométrique à une température comprise entre 50 et 200°C, de préférence comprise entre 80 et 120°C, en présence d'un catalyseur d'hydrogénation; (ii) l'on recycle une partie de l'effluent gazeux issu du réacteur comprenant du 1,1,1,2,3,3-hexafluoropropane (HFC-236ea), de l'hydrogène non réagi et éventuellement de l'hexafluoropropène non réagi, du 1,1,1,2,3-pentafluoropropane (HFC-245eb) et du 1,1,1,2-tetrafluoropropane (HFC-254eb) et (iii) l'on récupère du 1,1,1,2,3,3-hexafluoropropane de l'autre partie de l'effluent gazeux issu du réacteur éventuellement après une étape de purification.

De préférence, la température à l'entrée du réacteur est comprise entre 30 et 100° C, avantageusement comprise entre 40 et 80° C.

Le flux gazeux comprenant la boucle de recyclage et les réactifs peuvent être préchauffés avant introduction dans le réacteur.

Le procédé selon la présente invention est de préférence mis en oeuvre avec un rapport molaire hydrogène / HFP compris entre 1 et 50, avantageusement compris entre 2 et 15.

Le temps de contact, défini comme le rapport du volume du lit catalytique sur le débit volumique du flux total dans les conditions normales de température et de pression, est de préférence comprise entre 0,1s et 20s et avantageusement comprise entre 0,5 et 5s.

La réaction d'hydrogénation, selon la présente invention, est de préférence mise en oeuvre à une pression absolue comprise entre 0,5 et 20 bar et avantageusement comprise entre 1 et 5 bar.

L'effluent gazeux à la sortie du réacteur comprend de préférence environ 2 à 99 % en volume du 1,1,1,2,3,3-hexafluoropropane, 0,2 à 98% en volume d'hydrogène, 0 à 10 % du 1,1,1,2,3,3-hexafluoropropène, de 0 à 5% de 1,1,1,2,3 pentafluoropropane et de 0 à 1 % de 1,1,1,2-tetrafluoropropane.

Avantageusement, l'effluent gazeux à la sortie du réacteur comprend de 50 à 98 % en volume du 1,1,1,2,3,3-hexafluoropropane, 2 à 50% en volume d'hydrogène, 0 à 0,1% du 1,1,1,2,3,3-hexafluoropropène, de 0 à 1% de 1,1,1,2,3 pentafluoropropane et de 0 à 0,5% de 1,1,1,2-tetrafluoropropane.

Selon le procédé de l'invention on utilise, de préférence un réacteur adiabatique.

La partie de l'effluent gazeux recyclée au réacteur représente, de préférence au moins 90% en volume de la totalité de l'effluent à la sortie du réacteur, avantageusement au moins 93% en volume. De façon particulièrement préférée, la partie de l'effluent recyclée au réacteur représente entre 94 et 98% en volume de l'effluent total à la sortie du réacteur.

Comme catalyseur, on peut notamment citer ceux à base d'un métal du groupe VIII ou rhénium. Le catalyseur peut être supporté, par exemple sur carbone, alumine, fluorure d'aluminium, etc. ou peut ne pas être supporté, comme le nickel de Raney. Comme métal, on peut utiliser du platine ou du palladium, en particulier du palladium, avantageusement supporté sur carbone ou alumine. On peut aussi associer ce métal avec un autre métal comme l'argent, le cuivre, l'or, le tellure, le zinc, le chrome, le molybdène et le thallium.
De préférence, le catalyseur comprend du palladium éventuellement supporté.

Le catalyseur tout particulièrement préféré selon la présente invention est un catalyseur contenant du palladium supporté sur de l'alumine. La quantité de palladium dans le catalyseur est de préférence comprise entre 0,05 et 10% en poids et avantageusement entre 0,1 et 5%

La surface spécifique du catalyseur est de préférence supérieure à 4 m²/g et d l'alumine utilisé en tant que support catalytique se présente avantageusement sous la forme polymorphique α.

La demanderesse a remarqué de façon surprenante que la réactivité du 1,1,1,2,3,3-hexafluoropropane dans les conditions d'hydrogénation de la présente invention est très faible. Le procédé selon la présente invention permet d'atteindre une conversion de HFP supérieure à 99 %, voire 99,5% et même supérieure à 99,8% et une sélectivité en HFC-236ea supérieure à 99%, voire 99,5 et même supérieure à 99,8%. En outre, ces performances sont stables dans le temps.

### PARTIE EXPERIMENTALE

### Exemple 1

On utilise un réacteur tubulaire en inox de diamètre interne 2,1 cm et longueur 120 cm contenant 479 g, soit 330 cm3 de catalyseur sous forme d'un lit fixe. Le catalyseur contient 0,2 % en poids de palladium supporté sur alumine α.

Pendant la durée de la réaction, on injecte en continu 1,05 mol/h d'hydrogène et 0,7 mol/h de hexafluoropropène et le débit au sein de la boucle de recyclage est de 0,480 Nm3/h (représentant un taux de recyclage de 95,3 % en volume).

La pression est de 2 bar absolue. Le ratio molaire hydrogène/ HFP à l'entrée du réacteur est de 11,6 et la température en l'entrée de réacteur est de 31° C et la température maximale atteinte au cours de la réaction est de 121 ° C. Le temps de contact est de 2,28 s.

On obtient une conversion de 100% en HFP, une sélectivité de 99,39 % en HFC-236ea, de 0,53 % en HFC-245eb et de 0,07 % en HFC-254eb.

Aucune désactivation n'a été observée pendant 118h de fonctionnement.

### Exemple 2

On opère comme à l'exemple 1 sauf que la température à l'entrée du réacteur est de 81 °C et la température maximale atteinte au cours de la réaction est de 157,8° C.

On obtient une conversion de 100% en HFP, une sélectivité de 98,6 % en HFC-236ea, de 1,26 % en HFC-245eb et de 0,12 % en HFC-254eb.

### Exemple 3

On utilise un réacteur tubulaire en inox de diamètre interne 2,1 cm et longueur 120 cm contenant 479 g, soit 330 cm3 de catalyseur sous forme d'un lit fixe. Le catalyseur contient 0,2 % en poids de palladium supporté sur alumine α.

Pendant la durée de la réaction, on injecte en continu 0,84 mol/h d'hydrogène et 0,7 mol/h de hexafluoropropène et le débit au sein de la boucle de recyclage est de 0,480 Nm3/h (représentant un taux de recyclage de 96,2 % en volume).

La pression est de 2 bar absolue. Le ratio molaire hydrogène/ HFP à l'entrée du réacteur est de 6 et la température en l'entrée de réacteur est de 46,7° C et la température maximale atteinte au cours de la réaction est de 121,4° C. Le temps de contact est de 2,31 s.

On obtient une conversion de 100% en HFP, une sélectivité de 99,34 % en HFC-236ea, de 0,60 % en HFC-245eb et de 0,04 % en HFC-254eb.

### Exemple 4

On opère comme à l'exemple 3 sauf que l'on injecte en continu 1,4 mol/h d'hydrogène et le débit de la boucle de recyclage est de 93,9 %. Le ratio molaire hydrogène/ HFP à l'entrée du réacteur est de 17 et la température en l'entrée de réacteur est de 45,8° C et la température maximale atteinte au cours de la réaction est de 134,3° C.

On obtient une conversion de 100% en HFP, une sélectivité de 99,54 % en HFC-236ea, de 0,39 % en HFC-245eb et de 0,03 % en HFC-254eb.

## Revendications

1. Procédé de fabrication du 1,1,1,2,3,3-hexafluoropropane **caractérisé en ce que** (i) l'on fait réagir en phase gazeuse de l'hexafluoropropène avec de l'hydrogène en quantité surstoechiométrique à une température comprise entre 50 et 200 °C, en présence d'un catalyseur d'hydrogénation; (ii) l'on recycle une partie de l'effluent gazeux issu du réacteur comprenant du 1,1,1,2,3,3-hexafluoropropane, de l'hydrogène non réagi et (iii) l'on récupère du 1,1,1,2,3,3-hexafluoropropane de l'autre partie de l'effluent gazeux issu du réacteur éventuellement après une étape de purification.

2. Procédé selon la revendication 1 **caractérisé en ce que** la partie de l'effluent gazeux recyclé représente au moins 90 %, de préférence entre 94 et 98 % en volume de la totalité de l'effluent à la sortie du réacteur.

3. Procédé selon la revendication 1 ou 2 **caractérisé en ce que** l'effluent gazeux à la sortie du réacteur comprend de 2 à 99 % en volume du 1,1,1,2,3,3-hexafluoropropane, 0,2 à 98 % en volume d'hydrogène, de 0 à 10 % en volume de l'hexafluoropropène, de 0 à 1 % en 1,1,1,2-tetrafluoropropane et 0 à 5 % du 1,1,1,2,3-pentafluoropropane.

4. Procédé selon l'une quelconque des revendications précédentes **caractérisé en ce que** le catalyseur comprend du palladium, éventuellement supporté.

5. Procédé selon la revendication 4 **caractérisé en ce que** le support est à base d'alumine.

6. Procédé selon l'une quelconque des revendications précédentes **caractérisé en ce que** le rapport molaire hydrogène/hexafluoropropène est compris entre 1 et 50, de préférence compris entre 2 et 15.

7. Procédé selon l'une quelconque des revendications précédentes **caractérisé en ce que** le temps de contact est compris entre 0,1 s et 20 s et de préférence compris entre 0,5 et 5 s.

8. Procédé selon l'une quelconque des revendications précédentes **caractérisé en ce que** la réaction d'hydrogénation est mise en oeuvre à une pression comprise entre 0,5 et 20 bar absolu et de préférence comprise entre 1 et 5 bar absolu.

9. Procédé selon l'une quelconque des revendications précédentes **caractérisé en ce que** la réaction d'hydrogénation est mise en oeuvre en continu.

10. Procédé selon l'une quelconque des revendications précédentes **caractérisé en ce que** la température de réaction de l'étape (i) est comprise entre 80 et 120°C.

11. Procédé selon l'une quelconque des revendications précédentes **caractérisé en ce que** l'on recycle de l'héxafluoropropène non réagi, du 1,1,1,2,3-pentafluoropropane et du 1,1,1,2-tetrafluoropropane.

## Patentansprüche

1. Verfahren zur Herstellung von 1,1,1,2,3,3-Hexafluorpropan, **dadurch gekennzeichnet, dass** man (i) Hexafluorpropen in der Gasphase bei einer Temperatur zwischen 50 und 200°C in Gegenwart eines Hydrierkatalysators mit Wasserstoff in überstöchiometrischer Menge umsetzt; (ii) einen Teil des gasförmigen Austragsstroms aus dem Reaktor, der 1,1,1,2,3,3-Hexafluorpropan und nicht umgesetzten Wasserstoff umfasst, rezykliert und (iii) aus dem anderen Teil des gasförmigen Austragsstroms aus dem Reaktor 1,1,1,2,3,3-Hexafluorpropan gewinnt, gegebenenfalls nach einem Reinigungsschritt.

2. Verfahren nach Anspruch 1, **dadurch gekennzeichnet, dass** der rezyklierte Teil des gasförmigen Austragsstroms mindestens 90 Vol.-% und vorzugsweise zwischen 94 und 98 Vol.-% des gesamten Austragsstroms am Ausgang des Reaktors beträgt.

3. Verfahren nach Anspruch 1 oder 2, **dadurch gekennzeichnet, dass** der gasförmige Austragsstrom am Ausgang des Reaktors 2 bis 99 Vol.-% 1,1,1,2,3,3-Hexafluorpropan, 0,2 bis 98 Vol.-% Wasserstoff, 0 bis 10 Vol.-% Hexafluorpropen, 0 bis 1% 1,1,1,2-Tetrafluorpropan und 0 bis 5% 1,1,1,2,3-Pentafluorpropan umfasst.

4. Verfahren nach einem der vorhergehenden Ansprüche, **dadurch gekennzeichnet, dass** der Katalysator gegebenenfalls geträgertes Palladium umfasst.

5. Verfahren nach Anspruch 4, **dadurch gekennzeichnet, dass** der Träger auf Aluminiumoxid basiert.

6. Verfahren nach einem der vorhergehenden Ansprüche, **dadurch gekennzeichnet, dass** das Molverhältnis von Wasserstoff zu Hexafluorpropen zwischen 1 und 50 und vorzugsweise zwischen 2 und 15 liegt.

7. Verfahren nach einem der vorhergehenden Ansprüche, **dadurch gekennzeichnet, dass** die Kontaktzeit zwischen 0,1 s und 20 s und vorzugsweise zwischen 0,5 und 5 s liegt.

8. Verfahren nach einem der vorhergehenden Ansprüche, **dadurch gekennzeichnet, dass** die Hydrierungsreaktion bei einem Druck zwischen 0,5 und 20 bar absolut und vorzugsweise zwischen 1 und 5 bar absolut durchgeführt wird.

9. Verfahren nach einem der vorhergehenden Ansprüche, **dadurch gekennzeichnet, dass** die Hydrierungsreaktion kontinuierlich durchgeführt wird.

10. Verfahren nach einem der vorhergehenden Ansprüche, **dadurch gekennzeichnet, dass** die Reaktionstemperatur von Schritt (i) zwischen 80 und 120°C liegt.

11. Verfahren nach einem der vorhergehenden Ansprüche, **dadurch gekennzeichnet, dass** man das nicht umgesetzte Hexafluorpropen, 1,1,1,2,3-Pentafluorpropan und 1,1,1,2-Tetrafluorpropan rezykliert.

## Claims

1. Process for the manufacture of 1,1,1,2,3,3-hexafluoropropane, **characterized in that** (i) hexafluoropropene is reacted in the gas phase with hydrogen in a superstoichiometric amount at a temperature of between 50 and 200°C in the presence of a hydrogenation catalyst; (ii) a portion of the gaseous output stream resulting from the reactor, comprising 1,1,1,2,3,3-hexafluoropropane and unreacted hydrogen, is recycled and (iii) 1,1,1,2,3,3-hexafluoropropane is recovered from the other portion of the gaseous output stream resulting from the reactor, optionally after a purification stage.

2. Process according to Claim 1, **characterized in that** the portion of the gaseous output stream recycled represents at least 90% by volume, preferably between 94 and 98% by volume, of the whole of the output stream at the outlet of the reactor.

3. Process according to Claim 1 or 2, **characterized in that** the gaseous output stream at the outlet of the reactor comprises from 2 to 99% by volume of 1,1,1,2,3,3-hexafluoropropane, from 0.2 to 98% by volume of hydrogen, from 0 to 10% by volume of hexafluoropropene, from 0 to 1% of 1,1,1,2-tetrafluoropropane and from 0 to 5% of 1,1,1,2,3-pentafluoropropane.

4. Process according to any one of the preceding claims, **characterized in that** the catalyst comprises optionally supported palladium.

5. Process according to Claim 4, **characterized in that** the support is based on alumina.

6. Process according to any one of the preceding claims, **characterized in that** the hydrogen/hexafluoropropene molar ratio is between 1 and 50 and preferably between 2 and 15.

7. Process according to any one of the preceding claims, **characterized in that** the contact time is between 0.1 and 20 s and preferably between 0.5 and 5 s.

8. Process according to any one of the preceding claims, **characterized in that** the hydrogenation reaction is carried out at a pressure of between 0.5 and 20 bar absolute and preferably of between 1 and 5 bar absolute.

9. Process according to any one of the preceding claims, **characterized in that** the hydrogenation reaction is carried out continuously.

10. Process according to any one of the preceding claims, **characterized in that** the reaction temperature of stage (i) is between 80 and 120 °C.

11. Process according to any one of the preceding claims, **characterized in that** unreacted hexafluoropropene, 1,1,1,2,3-pentafluoropropane and 1,1,1,2-tetrafluoropropane are recycled.
